Europäisches Patentamt

European Patent Office

Office européen des brevets

(11) Veröffentlichungsnummer: **0 237 043 B1**

(12) **EUROPÄISCHE PATENTSCHRIFT**

(45) Veröffentlichungstag der Patentschrift: **03.06.92**

(51) Int. Cl.5: **A61L 27/00**

(21) Anmeldenummer: **87103483.1**

(22) Anmeldetag: **11.03.87**

Die Akte enthält technische Angaben, die nach dem Eingang der Anmeldung eingereicht wurden und die nicht in dieser Patentschrift enthalten sind.

(54) **Calciumphosphathaltiger, biokompatibler Schichtkörper und Verfahren zu seiner Herstellung.**

(30) Priorität: **14.03.86 DD 287917**

(43) Veröffentlichungstag der Anmeldung:
**16.09.87 Patentblatt 87/38**

(45) Bekanntmachung des Hinweises auf die Patenterteilung:
**03.06.92 Patentblatt 92/23**

(84) Benannte Vertragsstaaten:
**AT CH DE GB LI SE**

(56) Entgegenhaltungen:
**EP-A- 0 042 783**
**EP-A- 0 107 476**
**EP-A- 0 130 916**

(73) Patentinhaber: **Glasring Thüringen AG**
**Langewiesener Strasse 32**
**O-6300 Ilmenau(DE)**

(72) Erfinder: **Berger, Georg**
**Rosenthaler Strasse 5**
**O-1113 Berlin(DE)**
Erfinder: **Steinborn, Gabriele**
**Max-Koska-Strasse 5**
**O-1110 Berlin(DE)**
Erfinder: **Apel, Hans**
**J.-R.-Becher-Strasse 1**
**DDR-6325 Ilmenau(DE)**
Erfinder: **Dressel, Horst**
**W.-Pieck-Strasse 26**
**O-6325 Ilmenau(DE)**
Erfinder: **Hungenbach, Gudrun**
**Humboldtstrasse 63**
**O-6327 Ilmeneau(DE)**
Erfinder: **Leuner, Barbara**
**B.-Brecht-Strasse 8**
**O-6325 Ilmeneau(DE)**
Erfinder: **Katschner, Waltraud**
**Schwedter Strasse 51**
**O-1058 Berlin(DE)**
Erfinder: **Köhler, Steffen**
**Pfeilstrasse 2**
**O-1110 Berlin(DE)**
Erfinder: **Kunth, Peter Olaf**
**Gleimstrasse 10**
**O-1058 Berlin(DE)**
Erfinder: **Marx, Heidi**
**Bouchéstradse 37**
**O-1193 Berlin(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Sauer, Renate**
**Amalienstrasse 24**
**O-1120 Berlin(DE)**
Erfinder: **Schülke, Ulrich**
**Wolfsberger Strasse 36**
**O-1147 Berlin(DE)**
Erfinder: **Spitzer, Andrea**
**Altbuchhorster Strasse 13**
**O-1252 Berlin(DE)**


(74) Vertreter: **Patentanwälte Zellentin & Partner**
**Zweibrückenstrasse 15**
**W-8000 München 2(DE)**

## Beschreibung

Die Erfindung bezieht sich auf calciumphosphathaltige Materialien, und deren Herstellung, wobei diese Materialien direkt oder als Komposit- bzw. Verbundmaterial in verschiedenen Fachgebieten, insbesondere im Kontakt mit biologischen Milieu, wie z. B. beim Knochenersatz im Bereich der Humanmedizin, bei der Zellzüchtung in vitro etc angewendet werden können.

### Stand der Technik

Die einschlägige Patentliteratur läßt sich in verschiedene Gruppen gliedern:
1. Herstellung von Calciumphosphaten
2. Herstellung von Calciumphosphatkeramiken aus Tricalciumphosphat (TCP) und Hydroxylapatit (HA)
3. nachträgliche, die Calciumorthophosphatkeramik weiterverarbeitende bzw. -veredelnde Verfahrensschritte

Es sind in der wissenschaftlichen Literatur zahlreiche Verfahren beschrieben worden, nach denen Calciumorthophosphate derzeitig hergestellt werden können. Allerdings wurden diese Verfahren vielfältig verfeinert, um sie spezifischen Anwendungszwecken in optimaler Weise anzupassen. Beispielsweise werden in der US-PS 4 330 514 und 4 448 758 Verfahren beschrieben, nach denen frischgebranntes CaO unter Inertgas ($N_2$, Ar, He) gelöscht und mit $H_3PO_4$-Lösung zur Reaktion gebracht wurde. Ein Nachteil dieser verfeinerten Methode zur Herstellung einer Komposit-Komponente für die Implantatfertigung besteht zweifellos darin, daß alle Verfahrensschritte unter Inertgas durchgeführt werden müssen.

Die kontinuierliche Beschickung eines zweistufigen Reaktors mit CaO-Wasser-Schlamm und $H_3PO_4$ in US 4 324 772 führt lediglich zur Herstellung eines einheitlich aufgebauten Materials.

Diese Aussage trifft auch auf ein Material zu, hergestellt nach der GB-PS 1 586 915, die sich der Herstellung von Hydroxylapatit als "Synthetische Knochenasche" für die Knochenporzellanherstellung widmet. In diesem Fall wird zum Erhalt des Materials ein Schlickerstrom aus Kalkhydrat mit 60 %iger Phosphorsäure reagiert, analog der GB-PS 1 181 011, wobei es sich hier jedoch um ein Material handelt, das als Suspensionskatalysator zur Polymerisation von Styrol dient.

Bei der zweiten Gruppe (diese Verfahren gehen bereits von Calciumphosphaten aus, die z. B. nach Verfahren der o. g. Herstellungsarten erzeugt wurden), werden vielfach noch andere Komponenten vor dem Sintern hinzugefügt, wobei diese Komponenten dann im Prozeß des Sinterns chemisch mit dem Calciumphosphat-Ausgangsmaterial reagieren kann, wie in GB 1 487 181 und GB 1 548 066, oder sie können auch lediglich einen vorzugsweise physikalischen Verbund eingehen, wie in DE 3 301 122 beschrieben. Im letztgenannten Fall handelt es sich nämlich um eine Keramik aus einer Mischung von 70 - 95 % $TiO_2$, und lediglich der Rest wird durch ein Calciumorthophosphat, hier HA, gebildet.

Die Mehrzahl der Patentschriften der jüngsten Vergangenheit in der Gruppe 2 betrifft die Anwendung in der Humanmedizin, und hierbei wird auf die Herstellung eines porigen Produktes orientiert, wie es in EP 0 107 476 und DE 2 242 867 beschrieben wird. In diesen Fällen wird durch die oberflächliche porige Struktur mit einem gewissen besseren Calciumausstoß zu rechnen sein, jedoch birgt die porige Struktur, wie leicht einzusehen, erhebliche Nachteile bezüglich der mechanischen Beständigkeit in sich.

Andere Lösungen verweisen ausdrücklich auf die Ionen-Donator-Wirkung von Calciumionen (und/oder Magnesium-und/oder Kaliumionen), wie in DE 2 346 739, wo ein Sinterprodukt aus Apatit, Glas/Vitrokeram, Permutit zur Verwendung als Implantatmaterial oder fernerhin auch als biologisches Schadstofffilter beschrieben wird, führen jedoch zu Materialien, die hinsichtlich der Knochenneubildung noch immer nicht völlig befriedigend sind.

In der dritten Gruppe, die Calciumphosphatkeramiken betrifft, insbesondere unter dem Aspekt einer Verwendung des Materials in oder unter dem Einfluß des biologischen Milieus werden weiterverarbeitende bzw. die Materialien veredelnde Verfahrensschritte genannt, die nahezu ausnahmslos die Kompositbildung betreffen. Diese Erfindungen tangieren die vorliegende Beschreibung in keiner Weise. Lediglich in der DE 2 620 891 wird ein Komposit aus Calciumphosphat (CaO : $P_2O_5$ = 2:1 bis 4:1) und abbaubarem Polymer aufgeführt, das im Oberflächenbereich Calciumphosphat mit Porenstruktur besitzt, das aber wegen dieser porigen Struktur die bereits genannten Nachteile aufweisen muß.

Schichtartig aufgebaut ist ein Material, hergestellt nach DE 3 412 915, gebildet dadurch, daß unter Fliehkraftwirkung eine flüssige Ausgußmasse (flüssiges Aluminium oder Plaste) mit darin dispergierten HA-Teilchen zur Erstarrung gebracht wird, denn infolge unterschiedlicher Dichte befinden sich dann die Hydroxylapatit-Teilchen vorzugsweise an der Oberfläche der Körper.

Sowohl bei diesem als auch in der US-PS 4 314 380, ist keine wesentliche Veränderung der Calciumelution zu erwarten. Im letzterwähnten Falle wird nämlich denaturierter, von Tieren stammender Knochen, der ebenfalls als Calsiumorthophosphat-Formkörper aufgefaßt werden muß, mit Kollagen imprägniert und beschichtet bzw. mit Polyurethan imprägniert. In diesem Fällen ist sogar mit einer Dämpfung der Calciumfreisetzung zu rechnen.

Es ist ein Ziel der Erfindung, einen calciumphosphathaltigen Schichtkörper bereitzustellen, der mit einem biologischen Medium verträglich ist und nach Kontakt mit wäßrigen Lösungen eine erhöhte Calciumelution aufweist.

Ein weiteres Ziel ist es, die Stabilität des Schichtkörpers gegenüber seiner ursprünglichen Stabilität weitestgehend beizubehalten.

Ein weiteres Ziel ist es, die dem Material vorher zugehörigen günstigen Eigenschaften beizubehalten.

Ein weiteres Ziel ist es, ein Verfahren zur Erzeugung einer Schicht auf einem calciumphosphathaltigen Grundkörper bereitzustellen.

Ein weiteres Ziel ist es, Materialien bereitzustellen, die eine verbesserte Osteoneogenese als Implantat bewirken.

Wesen der Erfindung

Die Erfindung betrifft einen calciumphosphathaltigen, biokompatiblen Schichtkörper, der aus einem Grundkörper besteht, dessen Hauptbestandteil Calciumorthophosphat ist, und wobei besagter Schichtkörper wenigstens eine äußere, auf dem Grundkörper vorhandene Schicht aufweist, die Calciumionen abgibt, wobei die Calciumfreisetzung der Schicht höher ist als die Calciumfreisetzung des Grundkörpers. Der Grundkörper ist vorzugsweise ein Granulat oder ein kompakter Formkörper. Durch die verbesserte Calciumfreisetzung der Schicht gegenüber dem Grundkörper wird die Zellbildung in vivo oder in vitro besonders stark angeregt, und es kommt bei Verwendung dieses Materials z. B. zur Knochenhohlraumfüllung als resorbierbares Implantat zu einer sehr frühzeitigen Knochenneubildung (Osteoneogenese) oder auch bei langzeitstabilen Implantaten zu einer verbesserten Einheilung.

Das Herstellungsverfahren des Schichtkörpers besteht a) in einer Säurebehandlung des Grundkörpers oder in b) einem Überzug des Grundkörpers mit einer calciumhaltigen flüssigen Phase, deren Calciumfreisetzung über der des Grundkörpers liegt, sowie c) in einer Temperaturbehandlung bis etwa 1000 °C, der auf diese beiden Arten hergestellten Schicht.

Detaillierte Beschreibung der Erfindung

Der erfindungsgemäße Schichtkörper besitzt wenigstens eine äußere Schicht, deren Betrag der Calciumionenfreisetzung höher ist als der Betrag der Calciumionenfreisetzung des darunter liegenden Grundkörpers. Der Grundkörper besteht in der Hauptsache aus Calciumorthophosphat.

Unter "Calciumfreisetzung" oder "Calciumionenfreisetzung" wird im erfindungsgemäßen Zusammenhang die Eluierung von Calciumionen durch ein wäßriges Medium im Temperaturbereich von etwa 15 bis etwa 50 °C bei Normaldruck verstanden. Unter "Hauptbestandteil" im Grundkörper wird ein solcher Bestandteil verstanden, der mit wenigstens 10 Prozent an der Zusammensetzung der Gesamtmasse beteiligt ist.

Unter "Calciumorthophosphat" sind alle zwischen Calcium und Orthophosphorsäure ($H_3PO_4$) möglichen Verbindungen zu verstehen (s. a. Römpps Chemie-Lexikon, 8. Auflage, Bd. 1, S. 572).

Der Grundkörper ist vorzugsweise ein Granulat oder ein kompakter Formkörper, kann aber auch ein poröser Formkörper sein, wenn geringere Festigkeiten erforderlich sind.

Der Grundkörper kann weitere Komponenten enthalten, die entweder chemisch gebunden sind, oder er kann einen Gefügeaufbau haben, bei dem in eine Matrixphase eingebettete disperse Phasen vorliegen, z. B. durch unterschiedliche Kristallphasengehalte der Hoch- bzw. Tieftemperaturmodifikationen entsprechender Kristallphasen. Dabei muß jedoch mindestens eine Calciumorthophosphatphase vorliegen. Weitere Komponenten im Grundkörper können zum Beispiel Hydroxylapatit, Fluorapatit, Mischphosphate, β-Tricalciumphosphat-Mischkristalle usw. sein. Wenn beispielsweise im wesentlichen alpha-Tricalciumphosphat (alpha-TCP) das Material des Grundkörpers bildet und eine Behandlung mit Phosphorsäure erfolgt, erhält man ein Material, dessen Löslichkeit im biologischen Milieu erhöht ist und das sich für den Einsatz als resorbierbarer Implantatwerkstoff besonders eignet.

Die auf dem Grundkörper vorhandene Schicht bestcht vorzugsweise aus β-Calciumpyrophosphat oder Hydroxylapatit bei Behandlung von alpha-TCP oder HA respektive, mit Phosphorsäure.

Wenn man von einem im wesentlichen Hydroxylapatit enthaltenden Grundkörper ausgeht und mit Phosphorsäure behandelt, erhält man ein Material, dessen Löslichkeit (im biologischen Milieu) in der Oberflächenansicht erhöht ist, das dadurch in der Anfangsphase nach Implantierung besser einheilt, insgesamt jedoch ein langzeitstabiles Implantat mit entsprechend hoher Festigkeit darstellt.

Für die Erfindung ist wesentlich, daß sowohl der Grundkörper mit seinen möglichen darin enthaltenen Komponenten als auch die äußere Schicht biokompatibel ist, d. h. mit einem biologischen Medium wie Blut, Gewebe Knochen, interstitieller Körperflüssigkeit usw. verträglich ist.

Gemäß einer Ausführungsform der Erfindung kann die Schicht eine Mehrfachschicht sein, deren Einzelschichten eine chemisch abweichende Zusammensetzung aufweisen und deren Calciumfreisetzung in jeder Einzelschicht höher ist als die des Grundkörpers. Dabei kann jede Einzelschicht eine voneinander abweichende Calciumfreisetzung aufweisen. Das bedeutet, daß zum Beispiel die äußerste Schicht eine sehr hohe Calciumfreisetzung hat, eine darunter liegende zweite Schicht eine etwas niedrigere Calciumfreisetzung und eine dritte darunter liegende Schicht eine wiederum noch niedrigere Calciumfreisetzung aufweist, alle drei Schichten jedoch jede für sich eine höhere Calciumfreisetzung als die des unter der dritten Schicht liegenden Grundkörpers aufweisen. Ein solcher Calciumionengradient, der auch in umgekehrter Reihenfolge vorhanden sein kann, kann für besondere Zwecke sehr vorteilhaft sein.

Im allgemeinen wird davon ausgegangen, daß die Schicht nicht mehr als 1:1 Volumenanteile im Verhältnis zum Grundkörper einnimmt; bevorzugt nimmt die Schicht nicht mehr als 1 : 5 Volumenanteile im Verhältnis zum Grundkörper ein. Besonders bevorzugt wird eine Ausführungsform, bei der die Schicht (oder die Schichten) weniger als 1 : 10 Volumenanteile im Verhältnis zum Grundkörper einnimmt (einnehmen).

Der erfindungsgemäße Schichtkörper kann in oder unter Einwirkung eines biologischen Milieus verwendet werden und zeigt dabei eine erhöhte Abgabe von Calciumionen im Anfangsstadium, was insbesondere bei einem Knochenersatz von großer Bedeutung in der Einheilungsphase sein kann. Bei den nach herkömmlicher Art angesetzten Versuchen mit Agar-Kolonien wurde festgestellt, daß Humanknochenmarkzellen (HKM)-Kulturen einen sehr starken Wachstumimpuls in Gegenwart des erfindungsgemäßen Schichtkörpers aufweisen und keinerlei Toxizität vorliegt. Die stimulierende Wirkung auf die Zellproliferation wurde auf die in Lösung gehenden $Ca^{2+}$-Ionen zurückgeführt.

Die Erfindung besteht weiterhin in einem Verfahren zur Herstellung eines biokompatiblen schichtartig aufgebauten, calciumphosphathaltigen Körpers, das dadurch gekennzeichnet ist, daß

a) ein Calciumorthophosphat als Hauptbestandteil enthaltender Grundkörper der Einwirkung einer Säure über einen solchen Zeitraum ausgesetzt wird, daß die gebildete Schicht nicht mehr als 1:1 Volumenanteile im Verhältnis zum Grundkörper einnimmt, wobei eine solche Säure eingesetzt wird, deren Reaktionsprodukte mit dem Grundkörper eine höhere Calciumfreisetzung als die Calciumfreisetzung des Grundkörpers aufweisen; oder

b) ein Calciumorthophosphat als Hauptbestandteil enthaltender Grundkörper mit einer calciumhaltigen flüssigen Phase überzogen wird, deren Calciumfreisetzung über der des Calciumorthophosphates liegt, wobei die gebildete Schicht nicht mehr als 1 : 1 Volumenanteile im Verhältnis zum Grundkörper einnimmt; und

c) das gemäß a) oder b) hergestellte Material bei einer Temperatur im Bereich von etwa 400 °C bis etwa 1000 °C behandelt wird.

Der calciumorthophosphathaltige Grundkörper kann weitere Komponenten enthalten, die entweder chemisch gebunden sind, oder Komposite mit mindestens einer Calciumorthophosphatphase bilden.

Der Grundkörper ist vorzugsweise ein Granulat oder ein kompakter Formkörper, kann aber auch porös sein. Das Granulat sollte eine Größe von mindestens 63 $\mu$m haben. Bei einem Einsatz von beispielsweise als resorbierbares Implantat zur Füllung von Knochenhohlräumen (Zysten) hat sich ein Durchmesser von 100 bis 500 $\mu$m als zweckmäßig erwiesen. Bei einem Einsatz als langzeitstabiles Implantat, das wie bereits oben ausgeführt, im wesentlichen aus Hydroxylapatit bestehen kann, kann auch mit Granulaten gearbeitet werden, deren Durchmesser Millimetergröße aufweisen können. Granulate erhält man über übliche Granulierverfahren und durch Klassierung granulierter bzw. gemahlener Produkte. Derartige Granulierverfahren sind in Ullmann, Encyklopädie der technischen Chemie, Bd. 1, München 1951, S. 735 oder in Kirk-Othmer, Encyclopedia of chemical technology, Third Ed., Vol. 10, John Wiley & Sons, 1980, S. 84 ff. beschrieben.

Da es für die Erfindung wesentlich ist, ein biokompatibles Material zu erhalten, müssen entsprechende verdünnte Säuren vom Fachmann ausgewählt werden, deren Reaktionsprodukte biokompatibel sind. Zu diesen Säuren gehören beispielsweise Phosphorsäure, Schwefelsäure, Salzsäure, ein Gemisch aus Phosphor-, Schwefel- und Flußsäure oder auch organische Säure wie z. B. Citronensäure oder Milchsäure. Im Falle des Säure-gemisches sollte der Anteil an Flußsäure nicht höher als 5 % sein. Bevorzugt wird als Säure Phosphorsäure, insbesondere in einer Konzentration von 40 - 50 Masse-%. Diese Säure läßt man z. B. bei einer Temperatur von etwa 15 bis etwa 40 °C über einen Zeitraum von etwa 5 Minuten bis etwa 3 Stunden auf den Grundkörper einwirken. Allgemein kann die Temperatur bei jeder Art der Säurebehandlung des Grundkörpers im Bereich von 0 bis 100 °C liegen und ist vom Fachmann je nach Konzentration der Säure und gewünschter Schichtdicke auszuwählen. Die Schicht soll dabei nicht mehr als die Gleichen Volumenanteile wie der Grundkörper einehmen, was für den Fachmann durch Konzentration der Säure, Zeit der Einwirkung und Temperatur selbst bestimmt werden kann. Erhöhte Temperatur oder längere Einwirkzeit werden also eine größere Schichtdicke bewirken.

Die Temperaturbehandlung der erzeugten Schicht erfolgt in einem üblichen Ofen, dessen Temperatur im Bereich von ca. 300 bis ca. 1000 °C regelbar ist. Mit der Höhe der Temperatur wird die Reaktion der freien Säure mit dem Material des Grundkörpers gesteuert sowie eine Verfestigung der durch die Obenflächenbehandlung aufgelockerten Struktur.

Bei der Temperaturbehandlung eines nach Variante a) hergestellten Schichtkörpers können gewünschtenfalls Haltestufen eingelegt werden. Durch diese Haltestufen wird die Struktur der sich bildenden Reaktionsprodukte erzeugt, bzw. nachhaltig beeinflußt.

So kann z. B. bei 800 °C eine Haltestufe von beispielsweise 30 Minuten eingelegt werden, wodurch mit verdünnter Phosphorsäure behandeltes alpha-Tricalciumphosphat an der Oberfläche eine geschlossene Schicht ausbildet, die im wesentlichen $\beta$-Calciumpyrophosphat enthält.

Zu beachten ist dabei außerdem die Konzentration der angewandten Säure bzw. die Zusammensetzung der aufgebrachten Schicht. Im Falle der bevorzugten rein chemischen Erzeugung dieser Schicht werden Verteilungsprofile der Calciumionen erhöht freisetzenden Schicht in Form von diffusionsbedingten Formen vorhanden sein, d. h. mit allmählichem Abfall. Im Gegensatz dazu ist beim physikalischen Aufbringen (Verfahren b) der Schicht mit einem Kastenprofil, einem abrupten Abfall dieser Schicht zu rechnen. Die Aufbringung erfolgt hier beispielsweise durch Tauchen oder Besprühen. Zur besseren Haftung ist es im letzteren Fall sinnvoll, ebenfalls eine chemische Ankopplung im Grenzflächenbereich herbeizuführen. Bevorzugt ist das Aufbringen einer wäßrigen Suspension oder Lösung von Calciumpyrophosphat und/oder Calciumsulfat und/oder Calciumfluorid.

Die Erzeugung der gewünschten Schicht für Granulate und für kompakte Formkörper kann nach beiden Methoden erfolgen. Vorzugsweise auf die rein chemische Methode wird man dann zurückgreifen müssen, wenn es sich um ein Calciumorthophosphat enthaltenes Material mit offener Porenstruktur handelt. Dazu dient die Behandlung mit einer geeigneten Mineralsäure vorzugsweise Phosphorsäure, Schwefelsäure etc. Die offene Porenstruktur ermöglicht zusätzlich ein gutes Eindringen von z. B. Gewebe in das Material, wodurch die Zellbildung noch mehr beschleunigt wird und damit die Ziele der Erfindung noch besser erreicht werden.

Nach einer weiteren Ausführungsform der Erfindung können Mehrfachschichten durch nacheinanderfolgende Einwirkung verschiedener Säuren oder deren Gemischen unter Veränderung der Parameter: Zeit der Einwirkung und Temperatur und/oder Konzentration der Säuren erhalten werden. Im Rahmen dieser Bedingungen kann der Fachmann leicht zu der gewünschten Schichtenfolge kommen. So kann z. B. ein Granulat aus alpha-Tricalciumphosphat in einer 20 %igen Schwefelsäure über 10 Minuten bei 20 °C, anschließend in einem Gemisch aus 20 %iger Phosphorsäure, 20 %iger Schwefelsäure und 1 %iger Flußsäure über 15 Minuten bei 20 °C, und daran anschließend in einer 40 %igen Phosphorsäure über 30 Minuten bei 35 °C (behandelt werden). Nach dem Brennen bei z. B. 800 °C erhält man auf diese Weise einen Schichtkörper, der auf dem Grundkörper drei ineinander übergehende Schichten aufweist, deren Calciumfreisetzung von innen nach außen ansteigt (Gradient), wenn sie in ein wäßriges Medium eingebracht werden.

In ähnlicher Weise kann auch die Säurekonzentration mit der Zeit der Einwirkung ohne Temperaturveränderung kombiniert werden.

Diese allgemeinen Ausführungen, die lediglich den vollen Umfang der Erfindung erkennen lassen, sollen durch einige wenige Beispiele, die jedoch die Zielrichtung eindeutig markieren, anschaulicher und konkreter erläutert werden:

Beispiel 1 bis 5

Es wird ein alpha-Tricalciumphosphat-Granulat hergestellt. Diese Herstellung kann nach verschiedenen Verfahren erfolgen. Besonders vorteilhaft erwies sich jedoch die Verwendung von alpha-Tricalciumphosphat, das auf folgende Weise erzeugt wurde: Es wird eine Mischung aus Calciumcarbonat mit einer 85 %igen Phosphorsäure, die Calcium - zu Phosphorgesamtanteile entsprechend dem stöchiometrischen Verhältnis von Tricalciumphosphat aufweist, mit einem zusätzlichen Wasseranteil von ungefähr 10 Masse-% angeteigt und in geeigneter Weise homogenisiert, bei ca. 200 °C getrocknet und danach auf 1500 °C so lange erhitzt, bis röntgenographisch reines alpha-Tricalciumphosphat vorliegt. Das bei hohen Temperaturen erhaltene alpha-TCP wird abgeschreckt, damit keine Phasenumwandlung alpha-TCP, beta-TCP bzw. HA-Bildung stattfindet. Das Material wird entsprechend seiner spezifischen Anwendung granuliert. Dieses

EP 0 237 043 B1

Granulat wird einer weiteren Behandlung zur Erzeugung der bereits erwähnten Schicht unterzogen.

In eine verdünnte Phosphorsäurelösung wird das Material eingebracht und unterliegt eine gewisse Zeit dieser Einwirkung.

Sowohl durch die Wahl der Konzentration als auch durch die Behandlungsdauer wird maßgeblich die Art, d. h. der Chemismus, und die Dicke dieser erzeugten Schicht bestimmt.

Im Anschluß an diese Behandlung wird das Material erneut gebrannt. Der Brennprozeß kann in mehreren Stufen erfolgen und braucht teilweise bei Haltetemperatur nur 5 Minuten bis max. 3 Stunden lang zu erfolgen.

In Tabelle 1 sind einige Behandlungen und Anwendungsüberprüfungen angegeben.

## Tabelle 1

| Beispiel | 1 | 2 | 3 | 4 | 5 |
|---|---|---|---|---|---|
| Konzentration an $H_3PO_4$ /%/ | 40 | 10 | 40 | 5 | 10 |
| Behandlungs- dauer /h/ | 0,1 | 0,5 | 0,3 | 2 | 0,3 |
| Behandlungstempe- ratur /°C/ | 20 | 37 | 37 | 25 | 15 |
| Brenntempera- tur /°C/ | 1000 | 900 | 400,800 | 400,800 | 300,900 |
| Brenndauer /h/ | 1 | 1 | 1   1 | 1   0,1 | 0,2   1 |
| Anwendungsbe- reiche, die ge- testet wurden | Knochen- ersatz (tier.- exp.Über- prüfung | Zell- züch- tung | Zellzüch- tung, Knochen- ersatz (tier.exp. Überprüfung) (Einsatz i.d. Humanmedizin) | wie 1.1 | wie 1.1 |

Die genaue Wirkungsweise soll am Beispiel 7 noch näher erläutert werden, wozu Tab. 2 dient.

Tab. 2: Calcium-Elution eines unbehandelten und eines behan- delten Granulats entsprechend Ausführungsbeispiel 3
Versuchsbedingung: Granulat der Korngröße 315 - 500 /um, eluiert bei 80 °C,
in bidestilliertem Wasser, 1 h lang

## $Ca^{2+}$-Ionenkonzentration (mmol/l)

| | unbehandeltes Granulat | behandeltes Granulat |
|---|---|---|
| Probe I | 0,023 | 0,163 |
| Probe II | 0,028 | 0,222 |
| Probe III | 0,024 | 0,205 |
| Probe IV | 0,026 | 0,174 |

Die röntgenographische Untersuchung ergab, daß die gebildete Schicht mit dem Entstehen von geringen Mengen an HA und vor allem an $\beta$-Calciumpyrophosphat in Verbindung gebracht werden kann.

Beispiel 6:

Es wird ein Hydroxylapatitgranulat verwendet, das auf rein keramischem Wege durch geeignete Modifizierung des im Ausführungsbeispiel 1 beschriebenen Herstellungsweges oder auf dem Schmelzwege erhalten wurde, wobei hier die HA-Kristalle durch geringe Mengen einer Bindephase zusammengehalten werden (Komposit). Dieses Granulat (10 g) wird mit verdünnter, d. h. 40 %iger Phosphorsäure (100 ml) bei 25 °C behandelt und danach auf eine Temperatur von 800 °C erneut erhitzt, was schockartig erfolgen kann. Auch hier wird eine Schicht erzeugt, die insbesondere in der Anfangsphase des Kontaktes mit wäßriger Lösung, z. B. Blut, interstieller Körperflüssigkeit, Nährlösung etc., erhebliche Calciummengen freisetzt, und zwar etwa fünfmal mehr als der unbehandelte Hydroxylapatit. Das so behandelte Material wurde bislang tierexperimentell als Knochenersatzmaterial mit Vorteil eingesetzt.

Beispiel 7:

a) Es wurde ein mit 30 %iger Phosphorsäurelösung behandelter Formkörper, bestehend aus hochapatithaltigen glaskeramischem Material, eine bestimmte Zeit bei hoher Temperatur behandelt und danach 30 Minuten bei 850 °C erneut getempert.

b) Es wurde ein mit 30 %iger Phosphorsäurelösung behandelter Formkörper, der nur geringe Massenanteile apatitischer Materialien enthielt, die gleiche Zeit bei gleicher Temperatur wärmebehandelt und danach 50 Minuten unterhalb der höchsten Temperatur der Glaskeramik-Erzeugung, d. h. hier bei 920 °C, erneut getempert.

Sowohl im Falle a) als auch im Falle b) gelang es, eine Schicht zu erzeugen, die Calcium nach der Implantation in den Organismus von Tieren erhöht freisetzt, was aus in vitro-Untersuchungen geschlußfolgert wurde.

Die Wirkung zeigte sich im Tierexperiment so, daß eine schnellere Osteoneogenese erfolgte im Vergleich zu unbehandelten Proben.

Beispiel 8:

Analog Beispiel 7 wurden kompakte sinterglaskeramische Formkörper mit unterschiedlich hohem Gehalt an apatitischen Phasen mit 40 %iger Phosphorsäurelösung behandelt und nachfolgend bei 900 °C gebrannt. Das Charakteristikum dieser Formkörper war ferner, daß sie eine offene Porosität besaßen, die zum Zwecke einer biomechanischen Retention erzeugt worden waren. Auch hier gelang es, diese Schicht selbst im Innern der freien Oberfläche der Probekörper zu erzeugen. In diesem Falle war die Wirkung besonders eindrucksvoll, da das Material schneller durch Gewebe infiltriert wurde als jenes ohne diese Behandlung.

Beispiel 9 (Vergleichsbeispiel):

Ein Tricalciumphosphatgranulat der Korngröße 200 - 500 $\mu$m wurde 60 Minuten lang bei 37 °C in einer 5 %igen Schwefelsäurelösung gelagert und anschließend bei 400 °C gebrannt. Durch diese Behandlung wurde die Calcium-Freisetzung, wie in Tabelle 3 dargelegt, erfindungsgemäß erhöht.

**Tabelle 3: Calcium-Elution eines unbehandelten und eines behandelten Granulats entsprechend dem Ausführungsbeispiel 5**

Versuchsbedingungen: Granulat der Korngröße 200 – 500 $\mu$m eluiert bei 80 $^{o}$C, in bidestilliertes Wasser

| | $Ca^{2+}$-Ionen Konzentration (mmol/l) | |
|---|---|---|
| | unbehandeltes Granulat | behandeltes Granulat |
| Probe I | 0,07 | 1,73 |
| Probe II | 0,10 | 2,31 |
| Probe III | 0,08 | 1,81 |

Das Granulat wurde zur Knochenhohlraumfüllung im Tierexperiment - nach Überprüfung unter in vitro-Bedingungen - eingesetzt. Aucher hier wurde eine erhöhte Calciumfreisetzung in der postoperativen Anfangsphase beobachtet, wodurch offensichtlich die sehr früh einsetzende und umfassende Knochenneubildung impliziert wurde.

Obgleich die Erfindung im Hinblick auf ihre bevorzugten Ausführungsformen beschrieben wurde, insbesondere Beispiel 3 und 7, die die beste, den Erfindern gegenwärtig bekannte Art darstellen, sollte klar sein, daß zahlreiche Änderungen und Modifikationen für den auf dem Fachgebiet tätigen Fachmann naheliegend sind, ohne vom Schutzbereich der Erfindung, wie sie in den folgenden Patentansprüchen dargelegt ist, abzuweichen.

Beispiel 10 (Anwendungsbeispiel):

Bei einem 80jähringen Patienten mit stark reduziertem Lückengebiß und ausgedehnter Residualzyste im horizontalen Ast des rechten Unterkiefers (6x2,5 cm) erfolgte eine Extirpation der Zyste und Auffüllung des Defektes mit dem erfindungsgemäßen resorbierbaren, oberflächenbehandelten alpha-Tricalciumphosphat (Beispiel 3). Das Granulat wurde mit Fibrinkleber oberflächlich fixiert und die Wunde primär verschlossen.

Die Wundheilung verlief komplikationslos. Die p.o. Röntgenaufnahmen nach 6 Monaten und 18 Monaten zeigten eine Inkorporation des Materials mit deutlicher Knochenneubildung im Grenzbereich Knochen-Keramik. Die Form des Alveolarfortsatzes blieb erhalten und stellt eine gute Basis für die Prothesengestaltung dar.

Beispiel 11 (vergleichendes Anwendungsbeispiel):

a) Bei einer 43jährigen Patientin mit zwei follikulären Zysten im Kieferwinkelbereich ausgehend von 38 und 48 erfolgte eine Entoperation der Zyste 48, Extirpation und Auffüllung des Defektes mit unbehandeltem alpha-Tricalciumphosphat. Die p.o. Phase verlief komplikationslos. Die Röntgenkontrolle nach 2 Jahren zeigte nur geringe Hinweise auf eine Resorption des Materials.

b) Es erfolgte bei der gleichen Patientin eine Zweitoperation der Zyste bei 38, Extirpation und Auffüllung des Defektes mit granuliertem Material von Beispiel 3. Die Kontrolle nach einem halben Jahr zeigte eine deutlich stärkere Resorption als der Vergleichsdefekt bei 48 zum letzteren Kontrollzeitpunkt. Es zeigte sich weiterhin eine deutlich sichtbare Knochenneubildung.

**Patentansprüche**

1. Calciumphosphathaltiger, biocompatibler Schichtkörper, der aus einem Grundkörper aus ∝ -Tricalciumphosphat, Hydroxylapatit, Fluorapatit und $\beta$-Tricalciumphosphat oder Mischungen davon oder aus Calciumphosphathaltigem glasig-kristallinem Material besteht, und wenigstens einer äußeren auf dem Grundkörper befindlichen Schicht besteht, dadurch gekennzeichnet, daß die äußere Schicht aus einem oder mehreren Materialien der Gruppe Calciumpyrophosphat , Calciumsulfat oder Calciumfluorid, calciumhaltige Chloride oder einer Mischung mit dem Material des Grundkörpers besteht, wobei die Calciumfreisetzung der äußeren Schicht in biologischen Medien höher ist als die Calciumfreisetzung des Grundkörpers und die äußere Schicht weniger als 20 Vol.% ausmacht.

2. Schichtkörper nach Anspruch 1, dadurch gekennzeichnet, daß der Grundkörper neben Calciumorthophosphat in eine Matrix eingebettete disperse Phase enthält.

3. Schichtkörper nach Anspruch 1 oder 2, dadurch gekennzeichnet, daß die Schicht eine Mehrfachschicht ist, deren Einzelschichten eine chemisch abweichende Zusammensetzung aufweisen und deren Calciumfreisetzung in jeder Einzelschicht höher ist als die des Grundkörpers.

4. Schichtkörper nach einem der Ansprüche 1 bis 3, dadurch gekennzeichnet, daß der Grundkörper im wesentlichen aus alpha-Tricalciumphosphat und die äußere Schicht aus $\beta$-Calciumpyrophosphat besteht.

5. Schichtkörper nach einem der Ansprüche 1 bis 4, dadurch gekennzeichnet, daß die Schicht weniger als 10 Volumen-% im Verhältnis zum Grundkörper einnimmt.

6. Verfahren zur Herstellung eines Schichtkörpers gemäß einem der Ansprüche 1 - 5, dadurch gekennzeichnet, daß man einen Grundkörper der Zusammensetzung gemäß Anspruch 1
   a) der Einwirkung einer wäßrigen Säure aus der Gruppe, die aus Phosphorsäure, Schwefelsäure, Salzsäure oder dem Gemisch Phosphorsäure/-Schwefelsäure/Flußsäure besteht, bei einer Temperatur von etwa 15 bis etwa 40°C über einen Zeitraum von 5 Minuten bis 3 Stunden aussetzt, bis sich eine äußere Schicht, der Zusammensetzung und Dicke gemäß einem der Ansprüche 1 - 5, gebildet hat oder
   b) mit einer flüssigen Phase, ausgewählt aus der Gruppe wäßrige Suspension oder Lösung von Calciumpyrophosphat und/oder Calciumsulfat und/oder Calciumfluorid, überzogen wird, deren Calciumfreisetzung über der des Calciumorthophosphates liegt, wobei die gebildete Schicht nicht mehr als 1 : 1 Volumenanteile im Verhältnis zum Grundkörper einnimmt; und
   c) der gemäß a) oder b) hergestellte Schichtkörper zur Verfestigung der Schicht und/oder zur Umsetzung von noch freier Säure mit dem Grundkörper bei einer Temperatur im Bereich von etwa 400 °C bis etwa 1000 °C behandelt wird, wobei gegebenenfalls Haltestufen eingelegt werden.

7. Verfahren nach Anspruch 6, dadurch gekennzeichnet, daß man mehrere Schichten auf dem Grundkörper erzeugt, durch nacheinanderfolgende Einwirkung verschiedener Säuren unter Veränderung eines der Parameter Zeit der Einwirkung, Temperatur oder Konzentration der Mineralsäuren.

**Claims**

1. Biocompatible laminate containing calcium phosphate which consists of a base of ∝-tricalcium phosphate, hydroxyl apatite, fluoroapatite and $\beta$-tricalcium phosphate or mixtures thereof or of glass-crystalline material containing calcium phosphate, and consists of at least one outer layer situated on the base, characterized in that the outer layer consists of one or more materials of the group which includes calcium pyrophosphate, calcium phosphate or calcium fluoride, chlorides containing calcium or of a mixture with the material of the base, the calcium release of the outer layer in biological media being higher than the calcium release of the base, and the outer layer being less than 20% by volume.

2. Laminate according to claim 1, characterized in that the base contains as well as calcium phosphate a disperse phase embedded into a matrix.

3. Laminate according to either claim 1 or 2, characterized in that the layer is a multiple layer, the individual layers have a chemically divergent composition, and their calcium release in each individual layer is higher than that of the base.

4. Laminate according to one of claims 1 to 3, characterized in that the base consists substantially in alphatricalcium phosphate and the outer layer consists of betacalcium pyrophosphate.

5. Laminate according to one of claims 1 to 4, characterized in that the layer takes up less than 10% by volume in relation to the base.

6. A method of production of a laminate according to one of claims 1 to 5, characterized in that a base of composition according to claim 1

a) is subjected to the action of an aqueous acid of the group which consists of phosphoric acid, sulphuric acid, hydrochloric acid or a mixture of phosphoric acid, sulphuric acid/hydrofluoric acid, at a temperature of about 15 to about 40°C over a period of from 5 minutes to 3 hours, until the formation of an outer layer of composition and thickness according to one of claims 1 to 5, or

b) is coated with an aqueous phase, selected from the group of aqueous suspension or solution of calcium pyrophosphate and/or calcium phosphate and/or calcium fluoride, the calcium release of which is situated above that of the calcium orthophosphate, the layer formed taking up not more than 1 : 1 part by volume in relation to the base, and

c) the laminate produced according to a) or b) is treated for the solidification of the layer and/or for the reaction of acid having remained free with the base at a temperature within the range of form about 400°C to about 1000°C, in connection with which pause steps are possibly observed.

7. A method according to claim 6, characterized in that several layers are produced on the base by the succesive action of various acids, altering one of the parameters of action time, temperature or mineral acid concentration.

**Revendications**

1. Elément stratifié biocompatible, à teneur en phosphate de calcium, qui se compose d'un élément de base en phosphate tricalcique α, hydroxylapatite, fluorapatite et phosphate tricalcique β ou mélanges de ceux-ci ou en une matière vitreuse-cristalline à teneur en phosphate de calcium, et d'au moins une couche extérieure, se trouvant sur l'élément de base, caractérisé par le fait que la couche extérieure se compose d'une ou plusieurs matières du groupe du pyrophosphate de calcium, du sulfate de calcium ou du fluorure de calcium, des chlorures à teneur en calcium ou d'un mélange avec la matière de l'élément de base, 1a libération du calcium de la couche extérieure dans les milieux biologiques étant supérieure à la libération du calcium de l'élément de base, et la couche extérieure représentant moins de 20% en volume.

2. Elément stratifié selon la revendication 1, caractérisé par le fait que l'élément de base contient, en dehors de l'orthophosphate de calcium, une phase dispersée noyée dans une matrice.

3. Elément stratifié selon l'une quelconque des revendications 1 et 2, caractérisé par le fait que la couche est une couche multiple, dont les couches individuelles présentent une composition qui varie chimiquement, et dont la libération du calcium dans chaque couche individuelle est supérieure à celle de l'élément de base.

4. Elément stratifié selon l'une quelconque des revendications 1 à 3, caractérisé par le fait que l'élément de base se compose essentiellement de phosphate tricalcique alpha, et la couche extérieure de pyrophosphate de calcium ß.

5. Elément stratifié selon l'une quelconque des revendications 1 à 4, caractérisé par le fait que la couche extérieure représente moins de 10% en volume par rapport à l'élément de base.

6. Procédé de fabrication d'un élément stratifié tel que défini à l'une quelconque des revendications 1 à 5, caractérisé par le fait que l'on expose un élément de base ayant la composition telle définie à la revendication 1,

(a) à l'action d'un acide aqueux choisi dans le groupe qui se compose de l'acide phosphorique, de l'acide sulfurique, de l'acide chlorhydrique ou du mélange acide phosphorique/acide sulfurique/acide fluorhydrique, à une température d'environ 15 à environ 40°C, pendant un laps de temps de 5 minutes à 3 heures, jusqu'à ce qu'une couche extérieure, ayant la composition et l'épaisseur telles que définies à l'une quelconque des revendications 1 à 5, se soit formée;

(b) ou ledit élémant de base est recouvert par une phase liquide, choisie dans le groupe des suspensions ou solutions aqueuses de pyrophosphate de calcium/ou de sulfate de calcium et/ou de fluorure de calcium, dont la libération du calcium se situe au-delà de celle de l'orthophosphate de calcium, la couche formée ne représentant pas plus de 1 : 1 en parties en volume par rapport à l'élément de base ; et

(c) l'élément stratifié préparé selon (a) ou (b) est traité en vue du durcissement de la couche et/ou en vue de la réaction de l'acide encore libre avec l'élément de base à une température se situant dans la plage d'environ 400°C à environ 1000°C, des étapes d'arrêt étant, le cas échéant, intercalées

7.  Procédé selon la revendication 6, caractérisé par le fait que l'on forme plusieurs couches sur l'élément de base, par des actions successives de différents acides, avec modification de l'un des paramètres : temps de l'action, température ou concentration des acides minéraux.